# EUROPÄISCHE PATENTSCHRIFT

(11) **EP 2 782 507 B1**
(45) Veröffentlichungstag und Bekanntmachung des Hinweises auf die Patenterteilung: **11.01.2017**
(21) Anmeldenummer: 12818477.7
(22) Anmeldetag: 21.11.2012
(51) Int. Cl.: A61B 17/17

(54) **VERFAHREN ZUR HERSTELLUNG EINER VORRICHTUNG ZUR BOHRKANALPLATZIERUNG BEI DER FIXIERUNG KÜNSTLICHER BÄNDER AM KNOCHEN**
METHOD FOR PRODUCING A DEVICE FOR PLACING BORE CHANNELS WHEN ATTACHING ARTIFICIAL LIGAMENTS TO THE BONE
PROCÉDÉ DE PRODUCTION D'UN DISPOSITIF POUR PLACER UN CANAL DE PERÇAGE LORS DE LA FIXATION DE LIGAMENTS ARTIFICIELS SUR UN OS

(30) Priorität: 21.11.2011 DE 102011086717
(43) Veröffentlichungstag der Anmeldung: 01.10.2014
(73) Patentinhaber: Siebold, Rainer, 69190 Walldorf (DE)
(72) Erfinder: Siebold, Rainer, 69190 Walldorf (DE)
(74) Vertreter: Patent- und Rechtsanwälte Ullrich & Naumann
(86) Internationale Anmeldenummer: PCT/DE2012/200076
(87) Internationale Veröffentlichungsnummer: WO 2013/075711

(56) Entgegenhaltungen:
- US-A1- 2010 298 894

## Beschreibung

Die Erfindung betrifft ein Verfahren zur Herstellung einer Vorrichtung zur patientenspezifischen, individuellen anatomischen Bohrkanalplatzierung bei der Fixierung künstlicher Bänder am Ober- und Unterschenkel im Rahmen der vorderen Kreuzbandrekonstruktion.

Grundsätzlich betrifft die Erfindung die Herstellung eines maßangefertigten Instrumentariums zur patientenbezogenen individuellen anatomischen Bohrkanalplatzierung an menschlichen oder tierischen Knochen zur Fixierung künstlicher Bänder. Zur vereinfachten Erörterung der erfindungsgemäßen Lehre sei nachfolgend die Kreuzbandrekonstruktion beispielhaft erörtert, nämlich stellvertretend für jedwede patientenbezogene individuelle anatomische Bohrkanalplatzierung zur Bänderrekonstruktion.

Bei der Kreuzbandrekonstruktion werden am Ober- und Unterschenkel Bohrungen angebracht, um künstliche Kreuzbänder sicher zu verankern. Die anatomische Positionierung der Bohrkanäle am Ober- und Unterschenkel (Femur und Tibia) bei der vorderen Kreuzbandrekonstruktion ist von entscheidender Bedeutung für die einwandfreie biomechanische postoperative Kniegelenksfunktion. In Ermangelung eines hinreichend guten Kreuzband-Ziel-Instrumentariums treten nicht selten intraoperative Fehlplatzierungen des Bohrkanals bzw. der Bohrkanäle auf, und zwar von mehr als einem Zentimeter Fehlplatzierung. Solche Fehlplatzierungen führen entweder zu bleibenden Bewegungseinschränkungen, meist verbunden mit Schmerzen beim Patienten. Außerdem sind solche Fehlplatzierungen nicht selten Ursache zum "Ausleiern" der Kreuzbandrekonstruktion. Dabei droht die Gefahr einer Instabilität der Rekonstruktion mit zwangsläufigem Versagen nach gewisser Zeit. Oft sind schwierige und aufwändige Revisionsoperationen notwendig, wobei das Ergebnis immer schlechter ist als bei einer korrekt durchgeführten Erstoperation bei einwandfreier Bohrkanalplatzierung.

Voraussetzung für eine korrekte Bohrkanalplatzierung ist die genaue Kenntnis der Gelenkanatomie, insbesondere der Anatomie des Kreuzbandansatzes an Ober- und Unterschenkel sowie die Kenntnis verschiedener knöcherner Orientierungspunkte am Ober- und Unterschenkel. Nur dann lassen sich die aus der Praxis bekannten handelsüblichen Bohrkanal-Zielgeräte, meist ausgestattet mit einem punktartigen Spitzenzielarm, anatomisch genau platzieren. Bei allen aus der Praxis bekannten Zielgeräten ist es nachteilig, dass sie nicht nur das anatomische Wissen und ein genaues Platzieren des Zielgeräts voraussetzen, vielmehr universell ausgeformt sind, so dass bereits insoweit eine optimale Bohrkanalplatzierung - in Ermangelung einer individuellen Ausprägung des Zielgeräts - nicht möglich ist. Fehler sind daher vorprogrammiert, insbesondere unter Zugrundelegung der Tatsache, dass die meisten Kreuzbandrekonstruktionen von Operateuren durchgeführt werden, die pro Jahr nur wenige Kreuzbandrisse operieren.

Aus der US 2010/0298894 A1 ist eine Vorrichtung zur Bohrkanalplatzierung am Ober- und Unterschenkelknochen sowie ein Verfahren zur Herstellung einer entsprechenden Vorrichtung vorbekannt.

Der vorliegenden Erfindung liegt die Aufgabe zugrunde, ein Verfahren zur Herstellung einer entsprechenden Vorrichtung anzugeben.

Voranstehende Aufgabe ist durch die Merkmale der nebengeordneten Patentansprüche 1und 2 gelöst. Danach umfasst eine mit dem erfindungsgemäßen Verfahren hergestellte Vorrichtung folgende Merkmale:
Die Vorrichtung ist mit einer Sonde zum Einführen über ein Arthroskopieportal in ein Gelenk ausgestattet. Am distalen Ende der Sonde ist ein Zielkopf vorgesehen. Der Zielkopf umfasst eine individuell angefertigte Zielschablone, die unter Nutzung eines bildgebenden 3-D-Verfahrens, insbesondere der 3-D-Kernspintomographie oder der 3-D-Computertomographie, hergestellt worden ist. Im Konkreten handelt es sich bei der Zielschablone um einen Negativabdruck des knöchernen Oberflächenreliefs im Ansatzbereich des gerissenen Bandes am jeweiligen Knochen, insbesondere des Kreuzbandes am Oberschenkel und am Unterschenkel.

Mit anderen Worten handelt es sich bei der Vorrichtung um ein maßangefertigtes vorderes Kreuzband-Ziel-Instrumentarium zur patientenbezogenen individuellen anatomischen Bohrkanalplatzierung an Ober- und Unterschenkel auf der Grundlage einer präoperativen 3-D-Kernspintomographie oder einer 3-D-Computertomographie.

Die Zielschablone umfasst mindestens einen Durchgang zur Markierung des anzufertigenden Bohrkanals am Knochen, nämlich für die sog. Single-Bundle-Technik. Zur Realisierung der Double-Bundle-Technik umfasst die Zielschablone zwei Durchgänge zur Markierung von zwei Bohrkanälen am Knochen. In konstruktiver Hinsicht ist am proximalen Ende der Sonde ein Handgriff zum Positionieren vorgesehen, so dass in erfindungsgemäßer Weise ein handgehaltenes Zielgerät mit individuellem Zielkopf bzw. mit individueller Zielschablone geschaffen ist.

Das erfindungsgemäße Verfahren gemäß Ansprüchen 1 und 2 umfasst folgende Verfahrensschritte:
Durchführung einer 3-D-MRT oder einer 3-D-CT des verletzten Kniegelenks und des gesunden Kniegelenks zur Gewinnung von 3-D-Daten;
Erstellen eines 3D-Computermodells des verletzten Gelenks und des gesunden Gelenks mit dem jeweiligen Oberflächenprofil des individuellen Kreuzbandansatzes an Ober- und Unterschenkel unter Anwendung einer geeigneten Software;
Vergleich des 3-D-Computermodells des verletzten Kniegelenks und des 3-D-Computermodells des gesunden Kniegelenks,
Erzeugung individueller Zielschablonen und/oder Zielköpfe,
Herstellung der Vorrichtung.

Der Erfindung liegen neueste Erkenntnisse in der Kreuzbandchirurgie zugrunde, wonach ein größenadaptierter "maßgeschneiderter" vorderer Kreuzbandersatz realisiert werden soll. Dabei ist zu berücksichtigen, dass zwischen den Kniegelenken starke Größenunterschiede bestehen. Entsprechend haben kleinere Kniegelenke einen kleinen Kreuzbandansatz und umgekehrt. Die Größe des Kniegelenks korreliert nicht unbedingt mit der Größe der jeweiligen Person. Jedenfalls werden entsprechende Größenunterschiede in den Kniegelenken und bei der Operation bislang nicht individuell berücksichtigt. Eine solche individuelle Berücksichtigung ist unter Zugrundelegung der Vorrichtung, gefertigt nach dem erfindungsgemäßen Verfahren, in raffinierter Weise möglich.

Während die aus der Praxis bekannten Kreuzbandzielgeräte zur Anlage der Bohrkanäle am Oberschenkel und Schienbein keine eindeutige Hilfestellung für die korrekte Platzierung der Bohrkanäle liefern und obendrein die individuelle Größe der Kreuzbandinsertion am Oberschenkel und am Schienbein nicht berücksichtigen, ist mit der durch die erfindungsgemäßen Verfahren hergestellten Vorrichtung ein maßangefertigtes Kreuzband-Ziel-Instrumentarium angegeben, wonach eine individuelle Kreuzbandrekonstruktion möglich ist.

In vorteilhafter Weise ist die individuell gefertigte Zielschablone und ggf. der gesamte Zielkopf mit der Zielschablone am Instrument austauschbar.

Gemäß nebengeordnetem Anspruch 2 ist die Sonde mit dem Zielkopf und der Zielschablone Teil einer Fixiereinrichtung zum Fixieren der Sonde in der Kreuzbandregion oder mit einer entsprechenden Fixiereinrichtung kombinierbar. Ein solches Zielgerät wird mit der Sonde bspw. über ein Arthroskopieportal in das Gelenk eingeführt, wobei ein außerhalb des Gelenks befindlicher Teil des Zielgeräts bspw. am Schienbein passgerecht angelegt und positioniert wird.

An dieser Stelle sei angemerkt, dass die Erfindung die individuelle Anpassung einer Zielschablone bzw. eines Zielkopfes nutzt, und zwar ungeachtet des konkreten Geräts und ungeachtet der konkreten Technik, die zur Anwendung kommt. Wesentlich ist die Individualisierung der Zielschablone, um den anatomischen Gegebenheiten Rechnung zu tragen.

Die Fixiereinrichtung umfasst mindestens eine Führungshülse zur Definition der Bohrstelle und/oder des Bohrwinkels und/oder des Bohrdurchmessers und/oder zur Führung von Drähten, etc., nämlich zum Zwecke der Rekonstruktion. Eine optimale Operationshilfe ist geschaffen.

Im Lichte der voranstehenden Ausführungen wird deutlich, dass es mit Hilfe der durch die erfindungsgemäßen Verfahren hergestellten Vorrichtung möglich ist, für jeden Patienten zwei individuell angefertigte Zielschablonen und Zielgeräte bereitzustellen, nämlich jeweils für den Ober- und den Unterschenkel. Die Anfertigung erfolgte auf Basis einer 3-D-Kernspintomographie oder einer 3-D-Computertomographie, wobei es grundsätzlich möglich ist, jedwede bildgebende Verfahren zu verwenden. Mit Hilfe der durch die erfindungsgemäßen Verfahren hergestellten Vorrichtung wird die korrekte individuelle anatomische Bohrkanalplatzierung an Ober- und Unterschenkel bei der Kreuzbandrekonstruktion sichergestellt.

Durch Berücksichtigung der Größe des Kreuzbandansatzes an Ober- und Unterschenkel lässt sich mit Hilfe einer sog. Insertionstabelle eine ideale Größe der Bohrkanäle vorgeben. Die durch die erfindungsgemäßen Verfahren hergestellte Vorrichtung lässt sich dann sowohl für die Single-Bundle-Technik mit einem Bohrkanal als auch für die Double-Bundle-Technik mit zwei Bohrkanälen herstellen, so dass die Rekonstruktion der individuellen Länge/Fläche des Kreuzbandansatzes am Ober- und Unterschenkel für jeden Patienten individuell sichergestellt ist.

Die Herstellung der Vorrichtung mit der individuellen Zielschablone erfolgt unter Zugrundelegung der 3-D-Kernspintomographie oder der 3-D-Computertomographie am verletzten Kniegelenk und auch am gesunden Kniegelenk, um eine vergleichende Optimierung vornehmen zu können.

Mit Hilfe einer geeigneten Software wird ein digitales 3-D-Modell des verletzten Kniegelenks im Computermodell "gefertigt" bzw. wird ein 3-D-Modell simuliert. Dieses Modell zeigt das knöcherne Oberflächenprofil und die Ansätze des gerissenen Kreuzbandes am Ober- und Unterschenkel. Zur Darstellung der Kreuzbandstümpfe kann ein 3-D-Modell des gesunden Knies angefertigt werden, um einen zur Optimierung geeigneten Vergleich vornehmen zu können.

Unter Zugrundelegung des Computermodells lassen sich zwei digitale Zielschablonen für Ober- und Unterschenkel fertigen, um nämlich ein individuelles Zielgerät entsprechend ausstatten zu können. Die Zielschablonen sind letztendlich Negativabdrücke des tatsächlichen Oberflächenprofils und passen genau auf das individuelle Oberflächenrelief des jeweiligen Patienten im Ansatzbereich des Kreuzbandes an Ober- und Unterschenkel.

Auch ist es denkbar, unter Zugrundelegung einer geeigneten Software eine Simulation mit den individuell konzipierten Zielschablonen bzw. Zielgeräten am 3-D-Computermodell vorzunehmen, so dass vor der eigentlichen Fertigung der Zielschablonen eine Überprüfung der digitalen Vorlagen am kompletten Zielgerät möglich ist. In besonders vorteilhafter Weise lassen sich solche Kontrollen von dem zukünftigen Operateur mit Hilfe des 3-D-Modells vornehmen, so dass dieser die aus der Optimierung resultierende Funktionsfähigkeit überprüfen kann. Auch hat der Operateur die Möglichkeit, Korrekturen am 3-D-Modell vorzunehmen, nämlich zur Realisierung einer weiterreichenden Optimierung.

Auch ist es denkbar, interaktiv die Lokalisierung der Bohrkanäle, der Bohrwinkel, die Bohrkanalgröße sowie auch die durchzuführende Technik (Single-Bundle oder Double-Bundle) festzulegen, möglichst unter Zugrundelegung aus einer Datei abrufbarer Daten, bspw. unter Zugrundelegung von Daten aus einer Insertionstabelle. Eine manuelle Dateneingabe ist ebenso möglich.

Nach vollzogener Optimierung werden die patientenspezifischen Zielschablonen und ggf. Zielgeräte für Ober- und Unterschenkel auf der Basis des optimierten 3-D-Modells gefertigt, möglichst aus Metall oder aus Kunststoff. Beliebige formgebende Verfahren lassen sich anwenden, wobei die Fertigung unter Zugrundelegung der optimierten 3-D-Daten erfolgt.

Die so gefertigten Zielgeräte lassen sich passgerecht auf das knöcherne Oberflächenrelief des jeweiligen Patienten aufsetzen und dort justieren. Während der Operation werden mit Hilfe von integrierten Führungshülsen die Bohrer zum Erzeugen der Bohrkanäle angelegt oder die Kirschner-Drähte zum Durchführen durch die Bohrkanäle eingefädelt. Erst danach werden die Zielgeräte an Ober- und Unterschenkel entfernt.

Grundsätzlich ist es denkbar, dass die individuell gefertigte Zielschablone bzw. der individuell gefertigte Zielkopf auf handelsübliche Zielgeräte aufsetzbar ist, wobei die aus der Praxis bekannten Zielgeräte zum Ansetzen an den Unterschenkel aus zwei Teilen und die Zielgeräte für den Oberschenkel regelmäßig aus lediglich einem Teil bestehen kann.

Der operative Einsatz der erfindungsgemäßen Zielschablone und eines kompletten Zielgeräts vollzieht sich wie folgt:
Die individuelle tibiale Zielschablone (für den Unterschenkel) wird über ein Arthroskopieportal unter arthroskopischer Sicht passgerecht auf das knöcherne Oberflächenrelief aufgesetzt und dort justiert. Die Zielschablone wird von außen mit einem Handgriff gehalten. Dann können die Aussparungen der Bohrlöcher zur Bohrkanalmarkierung genutzt werden. Die Schablonen werden danach aus dem Gelenk entfernt und es wird an den markierten Stellen wie üblich gebohrt.

Das individuelle tibiale Zielgerät wird ebenfalls über ein Arthroskopieportal über das Gelenk eingeführt. Die Justierung erfolgt unter arthroskopischer Sicht auf das knöcherne Oberflächenprofil des Unterschenkels in der Kreuzbandregion. Der außerhalb des Gelenks liegende Teil des tibialen Zielgeräts wird an das Schienbein passgerecht angelegt. Damit definiert sich der Bohrwinkel am Schienbeinkopf quasi automatisch. Anhand des Designs des Zielgeräts wird entweder über eine übliche Single-Bundle-Technik oder über eine Double-Bundle-Technik unter Zugrundelegung der dem Zielkopf zugeordneten Führungshülsen der gewünschte Bohrkanaldurchmesser mit entsprechend vorgegebenem Winkel gebohrt. Kirschner-Drähte sind danach einbringbar. Grundsätzlich spielt die konkrete Technik keine Rolle, wonach nämlich entweder ein einziger Bohrkanal oder zwei Bohrkanäle gefertigt werden.

Am Unterschenkel lässt sich ebenfalls die durch die erfindungsgemäßen Verfahren hergestellte Vorrichtung bzw. die individuell gefertigte Zielschablone sowohl im Rahmen eines handgehaltenen Instruments als auch im Rahmen eines von außen anbringbaren Zielgeräts zum Einsatz bringen.

Die individuelle femurale Zielschablone (für den Oberschenkel) wird über ein Arthroskopieportal unter arthroskopischer Sicht passgerecht auf das knöcherne Oberflächenrelief aufgesetzt und dort justiert. Es wird von außen mit einem Handgriff gehalten. Dann können die Aussparungen der Bohrlöcher zur Bohrkanal-markierung genutzt werden. Die Schablonen werden danach aus dem Gelenk entfernt und es wird an den markierten Stellen wie üblich gebohrt.

Das individuelle femurale Zielgerät wird mit dem patientenspezifischen Abdruck der Oberflächenstruktur, d.h. mit der patientenspezifischen Schablone, in die Kreuzbandregion des Oberschenkels eingebracht, nämlich über ein anteromediales oder anterolaterales Arthroskopieportal. Das Instrument wird mit seinem passgenauen 3-D-Abdruck auf die knöchernen Strukturen der Kreuzbandregion unter arthroskopioscher Sicht am Oberschenkel im Bereich der lateralen Interkondylenwand aufgelegt. Gebohrt wird in diesem Falle über ein anteromediales Portal.

Alternativ kann das Instrument bzw. der Zielarm durch das anterolaterale Portal eingeführt werden. Ein sog. Outside-In-Bohren vom lateralen Oberschenkel her ist möglich.

Unter Zugrundelegung des individuellen Designs des Zielgeräts wird entsprechend einer oder zwei Führungshülsen zur Realisierung der Single-Bundle-Technik oder der Double-Bundle-Technik bei vorgegebenem Bohrkanaldurchmesser gebohrt. Kirschner-Drähte können durch die Bohrung(en) eingebracht werden.

Nach Anlage der Bohrkanäle wir das Transplantat wie üblich eingezogen und befestigt, nämlich unter Zugrundelegung einer individuellen Anlage der Bohrkanäle und unter Berücksichtigung patientenspezifischer Begebenheiten.

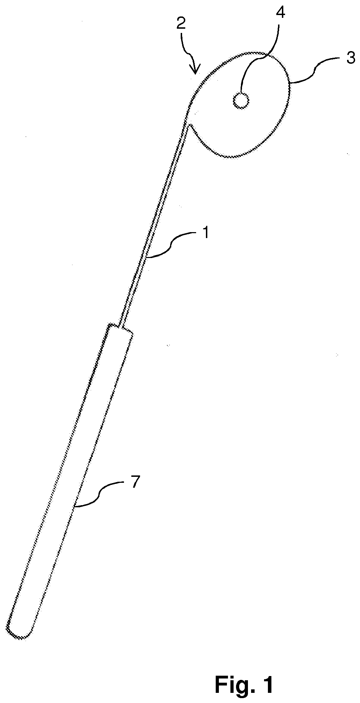

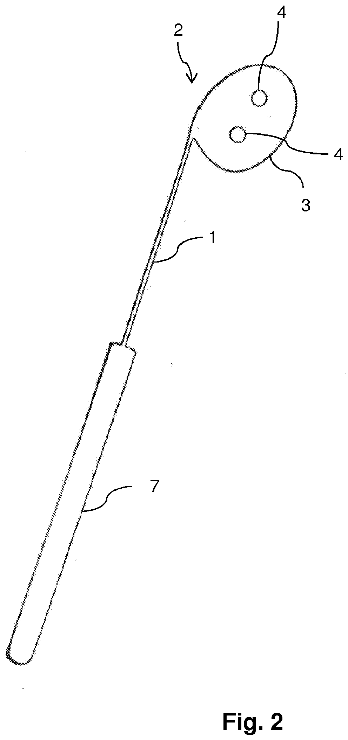

Es gibt nun verschiedene Möglichkeiten, die Lehre der vorliegenden Erfindung in vorteilhafter Weise auszugestalten und weiterzubilden. Dazu ist einerseits auf die den Ansprüchen 1 und 2 nachgeordneten Ansprüche und andererseits auf die nachfolgende Erläuterung bevorzugter Ausführungsbeispiele der Erfindung anhand der Zeichnung zu verweisen. In Verbindung mit der Erläuterung der bevorzugten Ausführungsbeispiele der Erfindung anhand der Zeichnung werden auch im Allgemeinen bevorzugte Ausgestaltungen und Weiterbildungen der Lehre erläutert. In der Zeichnung zeigen
- Fig. 1 und 2: in schematischen Ansichten zwei Ausführungsbeispiele einer durch die erfindungsgemäßen Verfahren hergestellten Vorrichtung am Beispiel eines handgehaltenen Geräts mit Handgriff, Sonde und Zielkopf, umfassend eine individuelle Zielschablone, einerseits zur Anwendung der Single-Bundle Technik (Fig. 1) und andererseits der Double-Bundle-Technik (Fig. 2), und

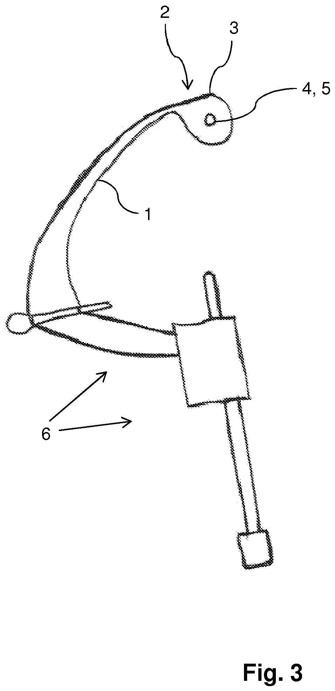

- Fig. 3: in einer schematischen Ansicht ein weiteres Ausführungsbeispiel einer durch die erfindungsgemäßen Verfahren hergestellten Vorrichtung, umfassend eine Sonde mit Zielkopf und individueller Zielschablone und einer Fixiereinrichtung zum Fixieren der Sonde in der Kreuzbandregion von außerhalb der Inzision.

Fig. 1 und 2 zeigen in schematischen Ansichten, andeutungsweise, eine durch die erfindungsgemäßen Verfahren hergestellte Vorrichtung zur patientenspezifischen, individuellen anatomischen Bohrkanalplatzierung am Ober- und Unterschenkel im Rahmen der vorderen Kreuzbandrekonstruktion.

Die Vorrichtung umfasst eine Sonde 1 zum Einführen über ein Arthroskopieportal in das Gelenk. Am distalen Ende der Sonde ist ein Zielkopf 2 vorgesehen. Dieser trägt eine individuell angefertigte Zielschablone 3, die unter Zugrundelegung einer 3-D-MRT oder einer 3-D-CT entsprechend den konkreten Gegebenheiten des jeweiligen Patienten gefertigt und in Vorbereitung der Fertigung optimiert worden ist. Fig. 1 zeigt im Konkreten eine Vorrichtung mit einem Durchgang 4 zur Realisierung der Single-Bundle-Technik, während Fig. 2 ein Ausführungsbeispiel der durch die erfindungsgemäßen Verfahren hergestellten Vorrichtung mit zwei Durchgängen 4 zeigt, nämlich zur Realisierung der Double-Bundle-Technik. Anstelle bloßer Durchgänge 4 können Führungshülsen 5 vorgesehen sein.

Fig. 3 zeigt ein weiteres Ausführungsbeispiel einer durch die erfindungsgemäßen Verfahren hergestellten Vorrichtung, wobei dort die Sonde 1 mit dem Zielkopf 2 und der Zielschablone 3 Teil einer Fixiereinrichtung 6 ist. Die Fixiereinrichtung 6 dient zur Positionierung bzw. Fixierung bspw. am Schienbein, während die Sonde 1 mit dem Zielkopf 2 und der Zielschablone 3 durch ein Portal in das Kniegelenk eingeführt wird, um dort passgenau zur Anlage zu kommen.

In Bezug auf weitere Merkmale der erfindungsgemäßen Lehre, die sich den Figuren nicht entnehmen lassen, sei zur Vermeidung von Wiederholungen auf den allgemeinen Teil der Beschreibung verwiesen.

Schließlich sei ausdrücklich darauf hingewiesen, dass die voranstehend beschriebenen Ausführungsbeispiele der durch die erfindungsgemäßen Verfahren hergestellten Vorrichtung lediglich zur Erörterung der beanspruchten Lehre dienen, diese jedoch nicht auf die Ausführungsbeispiele einschränken.

### Bezugszeichenliste

- 1: Sonde
- 2: Zielkopf
- 3: Zielschablone
- 4: Durchgang
- 5: Führungshülse
- 6: Fixiereinrichtung
- 7: Handgriff

## Patentansprüche

1. Verfahren zur Herstellung einer Vorrichtung zur patientenspezifischen, individuellen anatomischen Bohrkanalplatzierung bei der Fixierung künstlicher Bänder am Ober- und Unterschenkel im Rahmen der vorderen Kreuzbandrekonstruktion,
mit einer Sonde (1) zum Einführen über ein Arthroskopieportal in ein Gelenk und einem am distalen Ende der Sonde vorgesehenen Zielkopf (2),
wobei der Zielkopf (2) eine unter Nutzung eines bildgebenden 3-D-Verfahres individuell angefertigte Zielschablone (3) umfasst, die ein Negativabdruck des knöchernen Oberflächenreliefs im Ansatzbereich des gerissenen Kreuzbandes am Oberschenkel oder Unterschenkel ist, wobei die Zielschablone (3) mindestens einen Durchgang (4) zur Markierung des anzufertigenden Bohrkanals am Knochen umfasst und wobei am proximalen Ende der Sonde (1) ein Handgriff (7) zum Halten und Positionieren vorgesehen ist, mit folgenden Verfahrensschritten:
Durchführung einer 3-D-MRT oder einer 3-D-CT des verletzten Kniegelenks und des gesunden Kniegelenks zur Gewinnung von 3-D-Daten,
Erstellen eines 3D-Computermodells des verletzten Gelenks und des gesunden Gelenks mit dem jeweiligen Oberflächenprofil des individuellen Kreuzbandansatzes an Ober- und Unterschenkel unter Anwendung einer geeigneten Software,
Vergleich des 3-D-Computermodells des verletzten Kniegelenks und des 3-D-Computermodells des gesunden Kniegelenks,
Erzeugung individueller Zielschablonen (3) und/oder Zielköpfe (2), Herstellung der Vorrichtung.

2. Verfahren zur Herstellung einer Vorrichtung zur patientenspezifischen, individuellen anatomischen Bohrkanalplatzierung bei der Fixierung künstlicher Bänder am Ober- und Unterschenkel im Rahmen der vorderen Kreuzbandrekonstruktion,
mit einer Sonde (1) zum Einführen über ein Arthroskopieportal in ein Gelenk und einem am distalen Ende der Sonde vorgesehenen Zielkopf (2),
wobei der Zielkopf (2) eine unter Nutzung eines bildgebenden 3-D-Verfahrens individuell angefertigte Zielschablone (3) umfasst, die ein Negativabdruck des knöchernen Oberflächenreliefs im Ansatzbereich des gerissenen Kreuzbandes am Oberschenkel oder Unterschenkel ist, wobei die Sonde (1) mit dem Zielkopf (2) und der Zielschablone (3) Teil einer Fixiereinrichtung (6) zum Fixieren der Sonde (1) in der Kreuzbandregion ist oder mit einer entsprechenden Fixiereinrichtung (6) kombinierbar ist und wobei die Fixiereinrichtung (6) mindestens eine Führungshülse (5) zur Definition der Bohrstelle und/oder des Bohrwinkels und/oder des Bohrdurchmessers und/oder zur Führung von Drähten umfasst, mit folgenden Verfahrensschritten:
Durchführung einer 3-D-MRT oder einer 3-D-CT des verletzten Kniegelenks und des gesunden Kniegelenks zur Gewinnung von 3-D-Daten,
Erstellen eines 3D-Computermodells des verletzten Gelenks und des gesunden Gelenks mit dem jeweiligen Oberflächenprofil des individuellen Kreuzbandansatzes an Ober- und Unterschenkel unter Anwendung einer geeigneten Software,
Vergleich des 3-D-Computermodells des verletzten Kniegelenks und des 3-D-Computermodells des gesunden Kniegelenks,
Erzeugung individueller Zielschablonen (3) und/oder Zielköpfe (2), Herstellung der Vorrichtung.

3. Verfahren nach Anspruch 1 oder 2, **dadurch gekennzeichnet, dass** anhand der 3-D-Daten digitale, also virtuelle, Zielschablonen (3) erzeugt werden, anhand derer eine Simulation an vorgebbaren oder digital erzeugten, also virtuellen, Zielköpfen (2) und Zielgeräten durchführbar ist, um eine optimale Anpassung an das knöcherne Oberflächenrelief im Ansatzbereich des Kreuzbandes an Ober- und Unterschenkel, vor der Produktion der Zielschablone (3) bzw. des Zielkopfes (2) oder des Zielgeräts zu ermöglichen.

4. Verfahren nach Anspruch 3, **dadurch gekennzeichnet, dass** während oder nach der Simulation Korrekturen an den virtuellen Zielschablonen (3), Zielköpfen (2) und/oder Zielgeräten möglich ist.

5. Verfahren nach einem der Ansprüche 1 bis 4, **dadurch gekennzeichnet, dass** die Anpassung an das Oberflächenrelief unter Berücksichtigung eingebbarer oder aus einer Datei auswählbarer Parameter erfolgt.

6. Verfahren nach einem der Ansprüche 3 bis 5, **dadurch gekennzeichnet**, die Anpassung an das Oberflächenrelief unter Berücksichtigung der Lokalisation des Bohrkanals, des Bohrwinkels, der Bohrkanalgröße und der grundsätzlichen Technik, nämlich Single-Bundle oder Double-Bundle, erfolgt.

7. Verfahren nach einem der Ansprüche 1 bis 6, **dadurch gekennzeichnet, dass** die Zielschablonen, Zielköpfe und/oder Zielgeräte nach optimierter Anpassung unter Zugrundelegung der aus der Optimierung resultierenden Daten aus Metall oder Kunststoff gefertigt werden.

## Claims

1. Method for producing a device for patient-specific individual anatomical drill channel placement during the fixing of artificial ligaments to the upper and lower leg in the context of anterior cruciate ligament reconstruction, having a probe (1) for introduction via an arthroscopy portal into a joint and a target head (2) which is provided at the distal end of the probe,
wherein the target head (2) comprises a target template (3) which is individually produced using a 3D-imaging method and which is a negative image of the bone surface relief in the attachment region of the torn cruciate ligament to the upper leg or lower leg, wherein the target template (3) comprises at least one passage (4) for marking the drill channel which is intended to be produced on the bone and wherein at the proximal end of the probe (1) a handle (7) is provided for holding and positioning, having the following method steps:
carrying out a 3D MRT or a 3D CT of the injured knee joint and the healthy knee joint in order to obtain 3D data,
producing a 3D computer model of the injured joint and the healthy joint with the respective surface profile of the attachment of the individual cruciate ligament to the upper and lower leg using appropriate software,
comparing the 3D computer model of the injured knee joint and
the 3D computer model of the healthy knee joint,
producing individual target templates (3) and/or target heads (2),
producing the device.

2. Method for producing a device for patient-specific individual anatomical drill channel placement during the fixing of artificial ligaments to the upper and lower leg in the context of anterior cruciate ligament reconstruction, having a probe (1) for introduction via an arthroscopy portal into a joint and a target head (2) which is provided at the distal end of the probe,
wherein the target head (2) comprises a target template (3) which is individually produced using a 3D-imaging method and which is a negative image of the bone surface relief in the attachment region of the torn cruciate ligament to the upper leg or lower leg, wherein the probe (1) with the target head (2) and the target template (3) is part of a fixing device (6) for fixing the probe (1) in the cruciate ligament region or can be combined with a corresponding fixing device (6) and wherein the fixing device (6) comprises at least one guiding sleeve (6) for defining the drilling location and/or the drilling angle and/or the drill diameter and/or for guiding wires, having the following method steps:
carrying out a 3D MRT or a 3D CT of the injured knee joint and the healthy knee joint in order to obtain 3D data,
producing a 3D computer model of the injured joint and the healthy joint with the respective surface profile of the attachment of the individual cruciate ligament to the upper and lower leg using appropriate software,
comparing the 3D computer model of the injured knee joint and
the 3D computer model of the healthy knee joint,
producing individual target templates (3) and/or target heads (2),
producing the device.

3. Method according to claim 1 or claim 2, **characterised in that** there are produced using the 3D data digital, that is to say, virtual target templates (3), by means of which a simulation can be carried out on predeterminable or digitally produced, that is to say, virtual target heads (2) and target devices in order to enable optimum adaptation to the bone surface relief in the attachment region of the cruciate ligament to the upper and lower leg, before the production of the target template (3) or the target head (2) or the target device.

4. Method according to claim 3, **characterised in that**, during or after the simulation, corrections are possible on the virtual target templates (3), target heads (2) and/or target devices.

5. Method according to any one of claims 1 to 4, **characterised in that** the adaptation to the surface relief is carried out taking into account parameters which can be input or which can be selected from a file.

6. Method according to any one of claims 3 to 5, **characterised in that** the adaptation to the surface relief is carried out taking into account the localisation of the drill channel, the drill angle, the drill channel size and the technique in principle, that is to say, single bundle or double bundle.

7. Method according to any one of claims 1 to 6, **characterised in that** the target templates, target heads and/or target devices are produced in accordance with optimised adaptation from metal or plastics material taking as a basis the data resulting from the optimisation.

## Revendications

1. Procédé de fabrication d'un dispositif pour le positionnement, spécifique à un patient, individuel et anatomique d'un canal de perçage lors de la fixation de ligaments artificiels sur la cuisse et la jambe dans le cadre de la reconstruction des ligaments croisés postérieurs,
avec une sonde (1) destinée à être introduite, par l'intermédiaire d'un portail d'arthroscopie, dans une articulation et une tête cible (2) prévue au niveau de l'extrémité distale de la sonde,
la tête cible (2) comprenant un gabarit cible (3) fabriqué individuellement à l'aide d'un procédé 3D, qui est une empreinte négative du relief superficiel osseux dans la zone d'attache du ligament croisé déchiré au niveau de la cuisse ou de la jambe, le gabarit cible (3) comprenant au moins un passage (4) pour le marquage du canal de perçage à réaliser sur l'os et, au niveau de l'extrémité proximale de la sonde (1), une poignée (7) étant prévue pour le maintien et le positionnement, avec les étapes suivantes :
réalisation d'une IRM 3D ou d'une CT 3D de l'articulation de genou blessée et de l'articulation de genou saine pour l'acquisition de données 3D,
création d'un modèle informatique 3D du genou blessé et du genou sain avec le profil superficiel de l'attache individuelle du ligament croisé au niveau de la cuisse ou de la jambe à l'aide d'un logiciel approprié,
comparaison du modèle informatique 3D de l'articulation de genou blessée et du modèle informatique 3D de l'articulation de genou saine,
réalisation de gabarits cibles (3) et/ou de têtes cibles (2),
fabrication du dispositif.

2. Procédé de fabrication d'un dispositif pour le positionnement, spécifique à un patient, individuel et anatomique d'un canal de perçage lors de la fixation de ligaments artificiels sur la cuisse et la jambe dans le cadre de la reconstruction des ligaments croisés postérieurs,
avec une sonde (1) destinée à être introduite, par l'intermédiaire d'un portail d'arthroscopie, dans une articulation et une tête cible (2) prévue au niveau de l'extrémité distale de la sonde,
la tête cible (2) comprenant un gabarit cible (3) fabriqué individuellement à l'aide d'un procédé 3D, qui est une empreinte négative du relief superficiel osseux dans la zone d'attache du ligament croisé déchiré au niveau de la cuisse ou de la jambe, la sonde (1) faisant partie, avec la tête cible (2) et le gabarit cible (3), d'un dispositif de fixation (6) pour la fixation de la sonde (1) dans la zone des ligaments croisés ou pouvant être combinée avec un dispositif de fixation (6) correspondant et le dispositif de fixation (6) comprenant au moins un manchon de guidage (5) pour la définition de l'emplacement de perçage et/ou de l'angle de perçage et/ou du diamètre de perçage et/ou pour le guidage de fils, avec les étapes suivantes :
réalisation d'une IRM 3D ou d'une CT 3D de l'articulation de genou blessée et de l'articulation de genou saine pour l'acquisition de données 3D,
création d'un modèle informatique 3D du genou blessé et du genou sain avec le profil superficiel de l'attache individuelle du ligament croisé au niveau de la cuisse ou de la jambe à l'aide d'un logiciel approprié,
comparaison du modèle informatique 3D de l'articulation de genou blessée et du modèle informatique 3D de l'articulation de genou saine,
réalisation de gabarits cibles (3) et/ou de têtes cibles (2),
fabrication du dispositif.

3. Procédé selon la revendication 1 ou 2, **caractérisé en ce que**, à l'aide des données 3D, des gabarits cibles (3) numériques, donc virtuels, à l'aide desquels une simulation peut être réalisée sur des têtes cibles (2) et des appareils cibles pouvant être prédéfinis ou réalisés numériquement, donc virtuels, afin de permettre une adaptation optimale au relief superficiel osseux dans la zone d'approche du ligament croisé au niveau de la cuisse et de la jambe, avant la production du gabarit cible (3) ou de la tête cible (2) ou de l'appareil cible.

4. Procédé selon la revendication 3, **caractérisé en ce que**, pendant ou après la simulation, des corrections sont possibles sur les gabarits cibles (3), les têtes cibles (2) et/ou les appareils cibles virtuels.

5. Procédé selon l'une des revendications 1 à 4, **caractérisé en ce que** l'adaptation au relief superficiel a lieu en tenant compte de paramètres pouvant être saisis ou être sélectionnés dans un fichier.

6. Procédé selon l'une des revendications 3 à 5, **caractérisé en ce que** l'adaptation au relief superficiel a lieu en tenant compte de la localisation du canal de perçage, de l'angle de perçage, de la taille du canal de perçage et de la technique utilisée, à savoir « single-bundle » ou « double-bundle ».

7. Procédé selon l'une des revendications 1 à 6, **caractérisé en ce que** les gabarits cibles, les têtes cibles et/ou les appareils cibles sont fabriqués en métal ou en matière plastique avec une adaptation optimisée en tenant compte des données résultant de l'optimisation.
